# EUROPEAN PATENT SPECIFICATION

(11) **EP 3 860 528 B1**
(45) Date of publication and mention of the grant of the patent: **25.12.2024**
(21) Application number: 19791116.7
(22) Date of filing: 04.10.2019
(51) Int. Cl.: A61F 2/95

(54) **CONSTRAINING MECHANISMS**
BESCHRÄNKUNGSMECHANISMUS
MÉCANISMES DE CONTRAINTE

(30) Priority: 05.10.2018 US 201862741944 P
(43) Date of publication of application: 11.08.2021
(73) Proprietor: W. L. Gore & Associates, Inc., Newark, DE 19711 (US)
(72) Inventor: STASTKA, Jerry J., Newark, Delaware 19711 (US)
(74) Representative: HGF
(86) International application number: PCT/US2019/054630
(87) International publication number: WO 2020/072861

(56) References cited:
- EP-A1- 1 087 726
- EP-A1- 2 298 248
- EP-A1- 2 735 283
- EP-A1- 2 749 251
- JP-A- 2005 270 432
- US-A1- 2016 199 207
- US-B1- 6 302 891

## Description

### CROSS-REFERENCE TO RELATED APPLICATION

This application claims the benefit of Provisional Application No. 62/741,944, filed October 5, 2018.

### FIELD

The present disclosure relates to apparatuses and systems that include constructing coverings used in delivery of implantable medical devices. More specifically, the present disclosure relates to apparatuses and systems that include constructing coverings for constraining an expandable device during device delivery.

### BACKGROUND

Stents and stent-grafts may be utilized to radially support a variety of tubular passages in the body, including arteries, veins, airways, gastrointestinal tracts, and biliary tracts. The preferred method of placing these devices has been to use specialized delivery systems to precisely place and deploy a device at the site to be treated. These delivery systems allow the practitioner to minimize the trauma and technical difficulties associated with device placements. Attributes of delivery systems include: low profile; ability to pass through introducer sheaths; ability to negotiate tortuous vasculature, smoothly and atraumatically; protection of constrained devices; and ability to accurately position and deploy the device.

Stents or stent-grafts may be deployed and plastically deformed by using an inflatable balloon (*e.g*., balloon expandable stents) or to self-expand and elastically recover (*e.g*., self expandable devices) from a collapsed or constrained delivery diameter to an expanded and deployed diameter. Some stents are designed to elastically recover by being manufactured at their functional diameter out of a material that has elastic recovery properties, and then radially compressed to be mounted on a delivery catheter.

These stent and stent-graft devices may be held, compressed, or constrained in the delivery configuration prior to and during delivery to a target location. US2016199207 is directed to a stent delivery system that includes a self-expanding stent with a longitudinal length and a plurality of individual suture loops spaced along the longitudinal length of the stent, wherein the stent is compressed by the plurality of individual suture loops. The stent delivery system also includes a longitudinal pull member that secures the plurality of individual suture loops about the stent, wherein the plurality of individual suture loops are configured to separate from about the stent when the longitudinal pull wire is removed. EP2298248 is directed to a stent graft with an opening and a method for binding the stent graft. The stent graft includes multiple annular wave-shaped stent sections and a tectorial membrane pipe seamed with these stent sections. The tectorial membrane pipe (20) has at least one opening. There is an opening fixing wire seamed with the opening for supporting the opening. US6302891 is directed to a system-for repairing an anatomic duct, the system comprising an implant having a tubular body adapted to be radially expanded, once the implant is arranged in the duct and also a device for installing the implant in said duct. The device comprises a tie for maintaining the body in a restricted configuration, for the implantation thereof in the duct, and means for releasing the tie in order to allow the implant to radially expand. EP2749251 is directed to a system for deploying a stent-graft within a body vessel. The system includes an expandable stent-graft having expandable stent rings attached thereto and a cannula extending through the stent-graft. The system includes a plurality of diameter reducing connectors extending around the circumference of the graft for reducing the diameter of the graft. The system further includes a plurality of trigger wires extending longitudinally along the perimeter of the graft. The trigger wires extend through the diameter reducing connectors to constrain the connectors for reducing the diameter of the stent-graft. The trigger wires are also releasably attached to the apices of a bare stent portion extending from the proximal end of the graft body. The trigger wires can be sequentially retracted to release the bare stent and the diameter reducing connectors to expand the stent-graft radially into engagement with the wall of the body vessel. EP2735283 is directed to a temporary diameter reduction constraint arrangement for a stent graft. The arrangement comprises: primary and secondary release wires extending along the graft; a plurality of loops of thread, each loop engaged with either the primary or secondary wire and engaged around a portion of the graft circumferentially spaced away from its release wire, and drawn tight to reduce the diameter of the graft; an end constraint arrangement comprising four of the plurality of loops of thread arranged into a first and second pairs engaged with respective primary and secondary wires; and an intermediate constraint arrangement comprising a fifth and sixth of the plurality of loops of thread arranged into a third pair, the third pair engaged with the primary release wire, the primary release wire deviating towards the secondary release wire so as to locate the intermediate constraint arrangement substantially in-line with the end constraint arrangement. JP2005270432 is directed to a method of easily holding a stent in a reduced diameter state with a simple structure without requiring an outside sheath or a special device, and of easily and reliably controlling the expanded state of the stent. ;The device comprises a first loop-shaped part for inserting the stent in a reduced diameter state and a loop-shaped part for inserting a shaft are formed into a knot to be loosened by drawing in a wire. The stent in the reduced diameter state is inserted into the first loop-shaped part of the wire, and the shaft is inserted into the second loop-shaped part. Then, a second loop part for inserting the narrowed stent is formed as a knot to be loosened by drawing in the wire, and the stent in the reduced diameter state is inserted into the first loop-shape part of the wire, then the shaft is inserted into the second loop-shaped part. The above procedure is repeated a plurality of times from the distal end or near the distal end to the base end or near the base end on the opposite side.

### SUMMARY

The invention is defined by the appended claims.

Disclosed herein is a delivery system including an implantable medical device; a constraining mechanism including interlocking loops configured to releasably constrain the implantable medical device in a delivery configuration, and a lock line arranged through a portion of the interlocking loops and configured to be withdrawn to enable release of the constraining mechanism. The lock line comprises an adhesive on an exterior surface of the lock line to increase friction between the interlocking loops. In the delivery system, each loop of the interlocking loops includes at least two strands forming the loop, and wherein one strand of the loop overlaps with a strand of an adjacent loop to form an interlocking loop. The lock line is arranged in at least one of the knot rows to prevent the knot row from unraveling.

The interlocking loops may include at least two strands arranged in a warp knit having multiple knot rows spaced around a circumference of the implantable medical device.

The lock line may be arranged through one row of the interlocking loops.

The lock line may be arranged through both rows of the loops.

When one of the knot rows of the delivery system is disrupted, the constraining mechanism may unravel and may be remotely removable when a force is applied to a deployment line.

The lock line may comprise a linchpin.

The linchpin may be removable to allow a user to selectively unravel the at least one knot row.

The lock line may be configured to resist deployment when a ratio of a deployed diameter of the device to a delivery diameter of the device is less than 0.3.

The lock line may be arranged through each of the knot rows to allow for controlled release of each of the knot rows.

The lock line may be a first lock line, and wherein the system may further comprise a second lock line arranged through another portion of interlocking loops.

The second lock line may be spaced a distance from the first lock line around the circumference of the constraining mechanism.

The first lock line may be configured to release a first portion of the interlocking loops and the second lock line is configured to release a second portion of the interlocking loops.

Also disclosed but not claimed herein is a delivery system including an implantable medical device; a constraining mechanism configured to constrain the implantable medical device to a delivery configuration, and a lock line configured to increase friction between interlocking loops of at least one knot row to maintain the constraining mechanism in the delivery configuration.

The lock line may be removed from the delivery system by applying a force to the lock line, and wherein removal of the lock line releases the constraining mechanism.

Removal of the lock line may release each loop of the interlocking loops sequentially.

Applying the force to the lock line may decrease friction between the interlocking strands of the knot row to sequentially unravel the knot row.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a top plan view of a delivery system including a catheter with a constraining mechanism, in accordance with an embodiment;
FIG. 2 is a side view of an implantable medical device including a constraining mechanism, in accordance with an embodiment;
FIG. 3 is a schematic view of interlocking strands of the constraining mechanism, in accordance with an embodiment;
FIG. 4 is a schematic view of interlocking strands of the constraining mechanism, in accordance with an embodiment;
FIG. 5 is a schematic view of interlocking strands of the constraining mechanism having multiple lock lines, in accordance with an embodiment;
FIGS. 6A-6C are end views of the constraining mechanism showing example knot row positions, in accordance with an embodiment.
FIGS. 7A-7B are images of a delivery system in a delivery configuration and a semi-deployed configuration, respectively, in accordance with an embodiment;

As the terms are used herein with respect to ranges of measurements "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

While multiple examples are disclosed, still other embodiments will become apparent to those skilled in the art from the following detailed description, which shows and describes illustrative examples, as long as they are covered by the appended claims. Accordingly, the drawings and detailed description are to be regarded as illustrative in nature rather than restrictive in nature.

### DETAILED DESCRIPTION

Persons skilled in the art will readily appreciate that various aspects of the present disclosure can be realized by any number of apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, systems, and methods that include forming or manufacturing a constraining mechanism. The constraining mechanisms are configured to hold, compress, or constrain an implantable medical device (e.g., a stent, stent-graft, balloon, or other expandable medical device) in a delivery configuration prior to and during delivery to a target location. In certain instances, the constraining mechanism includes one or more fibers.

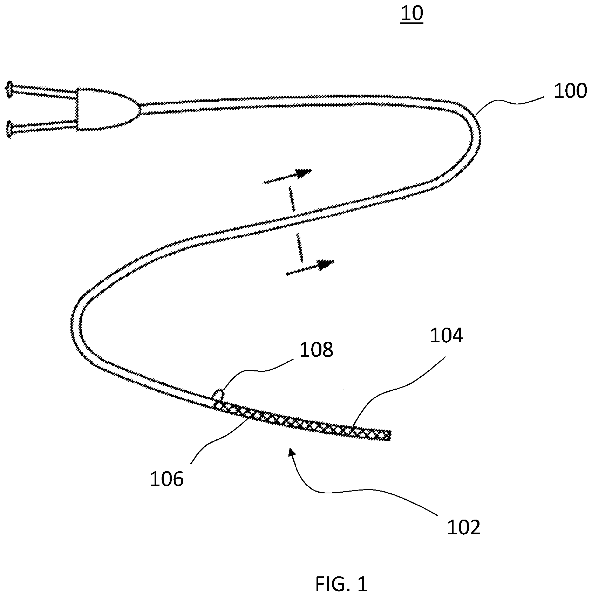

FIG. 1 is a top plan view of a delivery system 10 including a catheter 100 with a removable constraint 102, according to some embodiments. As shown in FIG. 1, the removable constraint 102 is configured to constrain an implantable medical device 104 to a delivery configuration. The removable constraint102 may include one or more fibers 106 arranged about the device 104 to maintain the removable constraint 102 in a constrained configuration.

The removable constraint 102 is arranged along a length of the device 104. The removable constraint 102 is also circumferentially arranged about the device 104 and may substantially cover the device 104 for delivery. The one or more fibers 106 may be arranged within a lumen (not shown) of the catheter 100 and extend toward a proximal end of the catheter 100 that is arranged external to a patient during delivery of the device 104. The one or more fibers 106 include a proximal end 108 that a user may apply tension to in order to release the removable constraint 102 and deploy the device 104.

In certain instances, the one or more fibers 106 release similar to a rip cord such that interlocking portions (e.g., overlapping fibers or knots) sequentially release along the length of the device 104. As is explained in greater detail below, the removable constraint 102 is formed by interlocking together the one or more fibers 106 directly on the device 104. The device 104 may be a stent, stent-graft, a balloon, or a similar device.

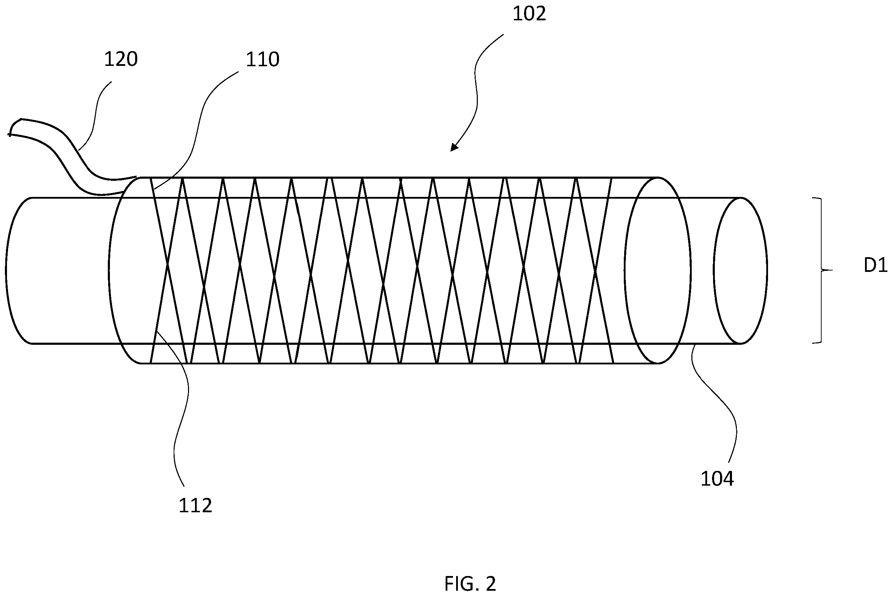

FIG. 2 is a side view of the device 104 including the constraining mechanism 102, in accordance with an embodiment. As shown, the device 104 includes a delivery diameter D1 and a deployed diameter D2 (not shown) that is larger than the delivery diameter D1. The removable constraint 102 is attached to the device 104 at its delivery diameter D1. As shown, the constraining mechanism 102 includes at least two interlocking strands in the form of a warp knit. For example, the constraining mechanism 102 includes a first interlocking strand 110 and a second interlocking strand 112. The constraining mechanism 102 includes a lock line 124 configured to release the constraining mechanism 102 and release the device 104 from the delivery diameter D1 to the deployed diameter D2 in response to a force applied to the lock line 124. In other terms, when the lock line 124 is removed from the constraining mechanism 102, the constraining mechanism 102 is released and decreases friction between the interlocking loops to sequentially unravel the at least one knot row.

The device 104 may have a desired deployed diameter D2 from about 5mm-15mm, or 6mm-9mm, or 6mm-12mm, for example, and a delivery diameter D1 that is less than the deployed diameter D2. For example, in some instances, a ratio of the delivery diameter D1 of the device 104 to the deployed diameter D2 (not shown) of the device 104 is less than about 0.3, less than about 0.29, less than about 0.28, less than about 0.27, or less than about 0.26. For reference, the term "diameter" is not meant to require a circular cross-section, and is instead to be understood broadly to reference a maximum transverse cross-sectional dimension of a device 104.

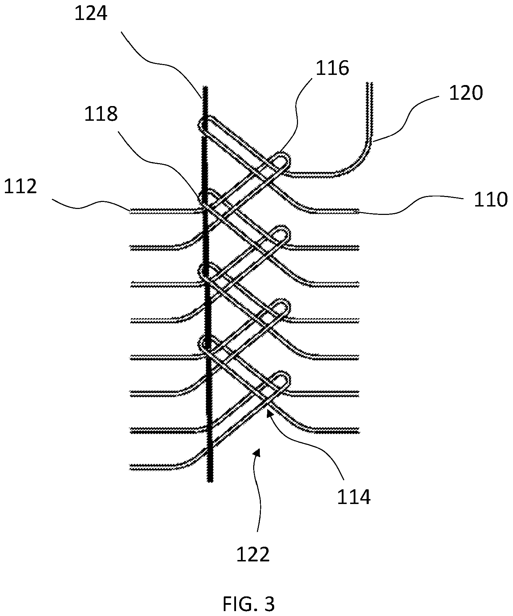

FIG. 3 is a schematic view of interlocking strands of the constraining mechanism 102, in accordance with an embodiment. The interlocking strands (e.g., the first and second interlocking strands 110, 112 as shown) are interwoven with one another to form at least one knot 114. As shown, the knot 114 is formed of interlocking loops formed from the first and second interlocking strands 110, 112. For example, the first interlocking loop 116 is formed by the first interlocking strand 110 and is interwoven with the second interlocking loop 118 formed by the second interlocking strand 112. This interlocking, looped configuration may be repeated to extend the longitudinal length of the device 104 (as shown in FIGs. 1, 2, and 7A-B) to form a knot row 122.

The lock line 124 is arranged through the knot row 122 of the constraining mechanism 102. The lock line 124, in connection with the interlocking strands 110, 112, is configured to lessen ramping (or deployment angle) of the device 104 prior to being released. For example, the lock line 124 may be configured to lessen ramping of the device 104 prior to the knots 114 being released in sequence. The device 104 begins to expand to a larger diameter after release of the constraining mechanism 102.

As discussed in further detail below, the lock line 124 lessens ramping of the device 104 (which may lead to uncontrolled or undesired deployment) by maintaining a location of each of the knots 114, relative to the device 104, as the knots 114 are released in sequence. The lock line 124, in this manner, lessens undesired or pre-deployment of the device. In some instances, the lock line 124 can be a fiber, wire, rod, or other similar device that is capable of extending along the knot row 122.

As force is applied to the lock line 124 and the lock line 124 is removed from the constraining mechanism 102, each of the knots 114 may be released in sequence. The knots 114 may be released as the lock line 124 is withdrawn or the knots 114 may be released by applying tension to a deployment line 120, which is an end of one or both of the interlocking strands 110, 112. Removal of the lock line 124 decreases friction between the interlocking strands of the constraining mechanism 102 to sequentially unravel the knot row 122. Thus, when force is applied to the lock line 124, the constraining mechanism 102 is remotely removable by a user.

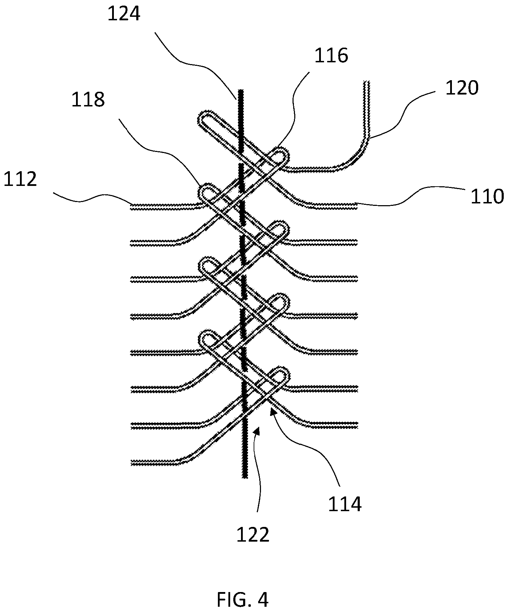

In some instances, the lock line 124 is arranged through one of the interlocking loops (e.g., either the first or second interlocking loop 116, 118), as shown in FIG. 3. In other instances, the lock line 124 can be arranged through both of the interlocking loops, as shown in FIG. 4. The lock line 124 is incorporated into at least one of the knots 114 to prevent the knot 114 from unraveling. Examples of suitable lock lines 124 can include linchpins, wires, metallic lines, fibers, and various adhesives. In certain instances, the lock line 124 is removable to allow a user to selectively unravel the respective knot 114. In some instances, the lock line 124 may be incorporated into each of the knots 114 for controlled release of all knots 114 of a respective knot row 122 (FIG. 3).

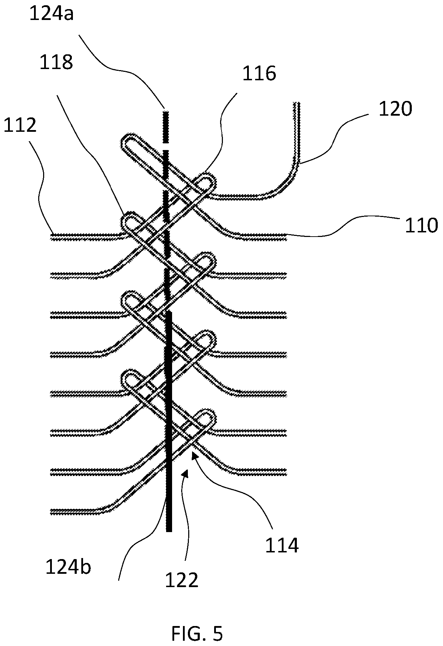

FIG. 5 is a schematic view of interlocking strands of the constraining mechanism 102 having multiple lock lines 124, in accordance with an embodiment. As shown, the knot row 122 can include more than one lock line 124. For example, the knot row 122 may include a first lock line 124a arranged through a first portion of the interlocking loops and a second lock line 124b arranged through a second portion of the interlocking loops. In certain instances, the first lock line 124a and the second lock line 124b longitudinally overlap. The first lock line 124a and the second lock line 124b may originate from a user end of the delivery system 10 that uses the constraining mechanism 102. In other instances, one of the first lock line 124a and the second lock line 124b may be coupled to or bifurcate from the other of the first lock line 124a and the second lock line 124b. The first lock line 124a may configured to release the first portion of the interlocking loops while the second lock line 124b is configured to release the second portion of interlocking loops. In some instances, the constraining mechanism 102 may include more than one knot row 122.

The knots 114 may be released as the lock lines 124a, 124b are withdrawn or the knots 114 may be released by applying tension to a deployment line 120, which is an end of one or both of the interlocking strands 110, 112. Removal of the lock lines 124a, 124b decreases friction between the interlocking strands of the constraining mechanism 102 to sequentially unravel the knot row 122. Thus, when force is applied to the lock line 124, the constraining mechanism 102 is remotely removable by a user.

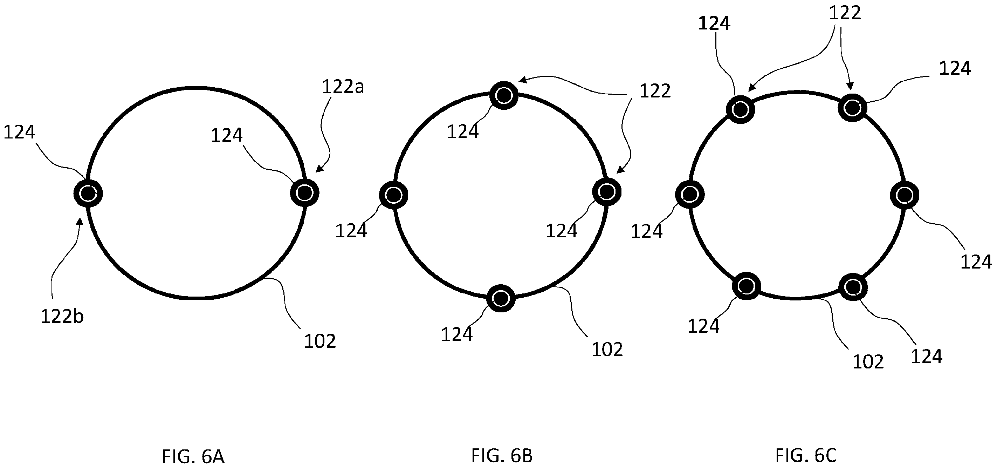

FIG. 6A is an end view of the device 104 including the constraining mechanism 102, according to an embodiment. As shown, the constraining mechanism 102 includes two knot rows. For example, the constraining mechanism 102 includes a first knot row 122a and a second knot row 122b spaced a distance from the first knot row 122a about the circumference of the constraining mechanism 102. In some instances, each of the knot row 122a, 122b includes a lock line 124 (e.g., a first lock line 124a and a second lock line 124b). For example, the first knot row 122a can include the first lock line 124a and the second knot row 122b can include the second lock line 124b.

FIGS. 6B and 6C are end views of the device 104 including the constraining mechanism 102, according to an embodiment. As shown, the constraining mechanism 102 can include more than two knot rows 122. For example, the constraining mechanism 102 can include four, six, or more knot rows 122 spaced about the circumference of the constraining mechanism 102 as desired. The loops in the knot rows 112 may be of different diameters in certain instances.

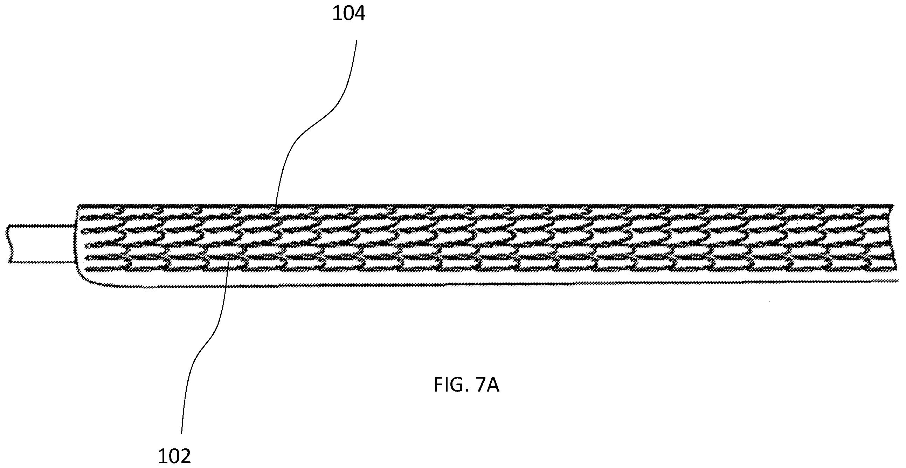

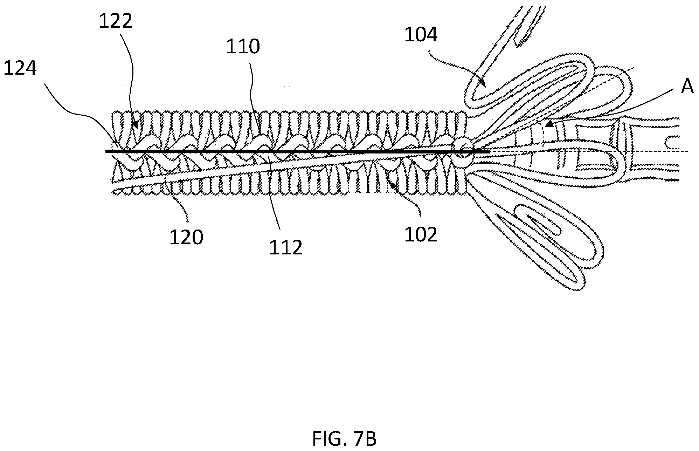

FIGS. 7A-7B are images of a delivery system in a delivery configuration and a semi-deployed configuration, respectively, in accordance with an embodiment. As shown in FIG. 7A, the removable constraint 102 is attached to the device 104 at its delivery diameter D1. During deployment, a force is applied to the lock line 124 to release the removable constraint 102 by unraveling the knot row 112, as shown in FIG. 7B. In certain instances, the knot row 112 may sequentially release, after the lock line 124 is removed, by applying a force to the deployment line 120. The device 104 is released to the deployed diameter D2 as the removable constraint 102 is released.

The lock line 124 can lessen ramping of the device 104 prior to being released. For example, the lock line 124 may lessen ramping of the device 104 prior to the knots of the knot row 122 being released in sequence. The device 104 begins to expand to a larger diameter after release of the constraining mechanism 102. The device 104 may be have an angle A between the portions held by the constraining mechanism 102 and portions that have been expanded or are beginning to expand. Due to the angle A and the device 104 expending a force to deploy to the deployed diameter D2, prior devices may shift due to ramping of the device 104. The lock line 124, however, resists spontaneous deployment that can be magnified by ramping of the device 104 with or without application of a radial force by the compressed stent.

The invention of this application has been described above both generically and with regard to specific embodiments. It will be apparent to those skilled in the art that various modifications and variations can be made in the embodiments.

## Claims

1. A delivery system (10) comprising:
an implantable medical device (104);
a constraining mechanism (102) including interlocking loops configured to releasably constrain the implantable medical device (104) in a delivery configuration, and
a lock line (124) arranged through a portion of the interlocking loops and configured to be withdrawn to enable release of the constraining mechanism (102), wherein the lock line (124) comprises an adhesive on an exterior surface of the lock line (124) to increase friction between the interlocking loops;
wherein each loop of the interlocking loops includes at least two strands (110, 112) forming the loop;
wherein one strand (110) of the loop overlaps with a strand (112) of an adjacent loop to form an interlocking loop (116, 118), and
wherein the lock line (124) is arranged in at least a knot row (122) to prevent the knot row (122) from unraveling.

2. The delivery system (10) of claim 1, wherein the interlocking loops include at least two strands (110, 112) arranged in a warp knit having multiple knot rows (122) spaced around a circumference of the implantable medical device (104).

3. The delivery system (10) of claim 2, wherein the lock line (124) is arranged through one row of the interlocking loops.

4. The delivery system (10) of claim 2, wherein the lock line (124) is arranged through both rows of the loops.

5. The delivery system (10) of any one of claims 2-4, wherein when one of the knot rows (122) is disrupted, the constraining mechanism (102) is configured to unravel and is remotely removable when a force is applied to a deployment line (120).

6. The delivery system (10) of any preceding claim, wherein the lock line (124) comprises a linchpin.

7. The delivery system (10) of claim 6, wherein the linchpin is removable to allow a user to selectively unravel the at least one knot row (122).

8. The delivery system (10) of claim 3 or 4, wherein the lock line (124) is configured to resist deployment when a ratio of a deployed diameter (D2) of the device to a delivery diameter (D1) of the device is less than 0.3.

9. The delivery system (10) of any preceding claim, wherein the lock line (124) is arranged through each of the knot rows (122) to allow for controlled release of each of the knot rows (122).

10. The delivery system (10) of any one of claims 1-9, wherein the lock line (124) is a first lock line (124a), and wherein the system (10) further comprises a second lock line (124b) arranged through another portion of interlocking loops.

11. The delivery system (10) of claim 10, wherein the second lock line (124b) is spaced a distance from the first lock line (124a) around the circumference of the constraining mechanism (102).

12. The delivery system (10) of claim 10, wherein the first lock line (124a) is configured to release a first portion of the interlocking loops and the second lock line (124b) is configured to release a second portion of the interlocking loops.

## Patentansprüche

1. Abgabesystem (10), umfassend:
eine implantierbare medizinische Vorrichtung (104);
einen Beschränkungsmechanismus (102), der ineinandergreifende Schlaufen beinhaltet, die dazu konfiguriert sind, die implantierbare medizinische Vorrichtung (104) in einer Abgabekonfiguration freigebbar zu beschränken, und
eine Verriegelungsleine (124), die durch einen Abschnitt der ineinandergreifenden Schlaufen hindurch angeordnet und dazu konfiguriert ist, herausgezogen zu werden, um die Freigabe des Beschränkungsmechanismus (102) zu ermöglichen, wobei die Verriegelungsleine (124) einen Klebstoff auf einer Außenfläche der Verriegelungsleine (124) umfasst, um die Reibung zwischen den ineinandergreifenden Schlaufen zu erhöhen;
wobei jede Schlaufe der ineinandergreifenden Schlaufen mindestens zwei Stränge (110, 112) beinhaltet, die die Schlaufe bilden;
wobei ein Strang (110) der Schlaufe mit einem Strang (112) einer benachbarten Schlaufe überlappt, um eine ineinandergreifende Schlaufe (116, 118) zu bilden, und
wobei die Verriegelungsleine (124) in mindestens einer Knotenreihe (122) angeordnet ist, um ein Auflösen der Knotenreihe (122) zu verhindern.

2. Abgabesystem (10) nach Anspruch 1, wobei die ineinandergreifenden Schlaufen mindestens zwei Stränge (110, 112) beinhalten, die in einem Kettstrickgewebe mit mehreren Knotenreihen (122) angeordnet sind, die um einen Umfang der implantierbaren medizinischen Vorrichtung (104) herum beabstandet sind.

3. Abgabesystem (10) nach Anspruch 2, wobei die Verriegelungsleine (124) durch eine Reihe der ineinandergreifenden Schlaufen hindurch angeordnet ist.

4. Abgabesystem (10) nach Anspruch 2, wobei die Verriegelungsleine (124) durch beide Reihen der Schlaufen hindurch angeordnet ist.

5. Abgabesystem (10) nach einem der Ansprüche 2-4, wobei, wenn eine der Knotenreihen (122) gestört wird, der Beschränkungsmechanismus (102) zum Auflösen konfiguriert ist und entfernt abnehmbar ist, wenn eine Kraft auf eine Entfaltungsleine (120) ausgeübt wird.

6. Abgabesystem (10) nach einem vorhergehenden Anspruch, wobei die Verriegelungsleine (124) ein Bindeglied umfasst.

7. Abgabesystem (10) nach Anspruch 6, wobei das Bindeglied abnehmbar ist, um einem Benutzer das selektive Auflösen der mindestens einen Knotenreihe (122) zu ermöglichen.

8. Abgabesystem (10) nach Anspruch 3 oder 4, wobei die Verriegelungsleine (124) dazu konfiguriert ist, dem Entfalten zu widerstehen, wenn ein Verhältnis eines entfalteten Durchmessers (D2) der Vorrichtung zu einem Abgabedurchmesser (D1) der Vorrichtung weniger als 0,3 beträgt.

9. Abgabesystem (10) nach einem vorhergehenden Anspruch, wobei die Verriegelungsleine (124) durch jede der Knotenreihen (122) hindurch angeordnet ist, um eine kontrollierte Freigabe jeder der Knotenreihen (122) zu ermöglichen.

10. Abgabesystem (10) nach einem der Ansprüche 1-9, wobei die Verriegelungsleine (124) eine erste Verriegelungsleine (124a) ist und wobei das System (10) ferner eine zweite Verriegelungsleine (124b) umfasst, die durch einen anderen Abschnitt ineinandergreifender Schlaufen hindurch angeordnet ist.

11. Abgabesystem (10) nach Anspruch 10, wobei die zweite Verriegelungsleine (124b) in einer Entfernung von der ersten Verriegelungsleine (124a) um den Umfang des Beschränkungsmechanismus (102) herum beabstandet ist.

12. Abgabesystem (10) nach Anspruch 10, wobei die erste Verriegelungsleine (124a) dazu konfiguriert ist, einen ersten Abschnitt der ineinandergreifenden Schlaufen freizugeben, und die zweite Verriegelungsleine (124b) dazu konfiguriert ist, einen zweiten Abschnitt der ineinandergreifenden Schlaufen freizugeben.

## Revendications

1. Système de pose (10) comprenant :
un dispositif médical implantable (104) ;
un mécanisme de contrainte (102) comprenant des boucles d'interverrouillage conçues pour contraindre de manière libérable le dispositif médical implantable (104) dans une configuration de pose, et
une ligne de verrouillage (124) disposée à travers une partie des boucles d'interverrouillage et conçue pour être retirée de manière à permettre la libération du mécanisme de contrainte (102), ladite ligne de verrouillage (124) comprenant un adhésif sur une surface externe de la ligne de verrouillage (124) destiné à augmenter le frottement entre les boucles d'interverrouillage ;
chaque boucle des boucles d'interverrouillage comprenant au moins deux brins (110, 112) formant la boucle ;
un brin (110) de la boucle chevauchant un brin (112) d'une boucle adjacente de manière à former une boucle d'interverrouillage (116, 118), et
ladite ligne de verrouillage (124) étant disposée dans au moins une rangée de noeuds (122) de manière à empêcher la rangée de noeuds (122) de se dénouer.

2. Système de pose (10) selon la revendication 1, lesdites boucles d'interverrouillage comprenant au moins deux brins (110, 112) disposés dans un tricot-chaîne comportant plusieurs rangées de noeuds (122) espacées autour d'une circonférence du dispositif médical implantable (104).

3. Système de pose (10) selon la revendication 2, ladite ligne de verrouillage (124) étant disposée à travers une rangée de boucles d'interverrouillage.

4. Système de pose (10) selon la revendication 2, ladite ligne de verrouillage (124) étant disposée à travers les deux rangées des boucles.

5. Système de pose (10) selon l'une quelconque des revendications 2 à 4, lorsque l'une des rangées de noeuds (122) est interrompue, ledit mécanisme de contrainte (102) étant conçu pour se dénouer et étant amovible à distance lorsqu'une force est appliquée sur une ligne de déploiement (120).

6. Système de pose (10) selon l'une quelconque des revendications précédentes, ladite ligne de verrouillage (124) comprenant une goupille.

7. Système de pose (10) selon la revendication 6, ladite goupille étant amovible de manière à permettre à un utilisateur de dénouer de manière sélective ladite au moins une rangée de noeuds (122).

8. Système de pose (10) selon la revendication 3 ou 4, ladite ligne de verrouillage (124) étant conçue pour résister au déploiement lorsqu'un rapport entre un diamètre déployé (D2) du dispositif et un diamètre de pose (D1) du dispositif est inférieur à 0,3.

9. Système de pose (10) selon l'une quelconque des revendications précédentes, ladite ligne de verrouillage (124) étant disposée à travers chacune des rangées de noeuds (122) de manière à permettre une libération contrôlée de chacune des rangées de noeuds (122).

10. Système de pose (10) selon l'une quelconque des revendications 1 à 9, ladite ligne de verrouillage (124) étant une première ligne de verrouillage (124a), et ledit système (10) comprenant en outre une seconde ligne de verrouillage (124b) disposée à travers une autre partie des boucles d'interverrouillage.

11. Système de pose (10) selon la revendication 10, ladite seconde ligne de verrouillage (124b) étant espacée d'une certaine distance de la première ligne de verrouillage (124a) autour de la circonférence du mécanisme de contrainte (102).

12. Système de pose (10) selon la revendication 10, ladite première ligne de verrouillage (124a) étant conçue pour libérer une première partie des boucles d'interverrouillage et ladite seconde ligne de verrouillage (124b) étant conçue pour libérer une seconde partie des boucles d'interverrouillage.
